# EUROPEAN PATENT APPLICATION

(11) **EP 4 679 119 A1**
(43) Date of publication of application: **14.01.2026**
(21) Application number: 24187910.5
(22) Date of filing: 11.07.2024
(51) Int. Cl.: G01R 33/28, A61B 6/03, A61B 5/00, A61B 6/00

(54) **MEDICAL IMAGING SYSTEM WITH OPTIMIZED REMOTE PATIENT SENSING**

(71) Applicant: Koninklijke Philips N.V., 5656 AG Eindhoven (NL)
(72) Inventor: KRUEGER, Sascha, Eindhoven (NL); WEIß, Steffen, Eindhoven (NL); WUELBERN, Jan Hendrik, 5656AG Eindhoven (NL); POSSANZINI, Cecilia, Eindhoven (NL)
(74) Representative: Philips Intellectual Property & Standards

(57) **Abstract**

It is presented a medical imaging system (1) comprising: a medical imaging device (2) comprising an examination bore (3), a patient support (5), wherein the patient support (5) is movable between an in-bore and an out-bore position, a workflow camera system (7) arranged outside the examination bore (3) and adapted to monitor a patient support area (11) outside the examination bore (3), a reflection system (9) comprising at least one reflective surface (19), wherein the reflection system (9) is adapted to provide a line-of-sight (13) between an area of interest (15) and an external device (17) arrangeable remotely to the area of interest (15) to emit radiation to the area of interest (15) and/or receive radiation from the area of interest (15) during examination, wherein the workflow camera system (7) is adapted to detect an arrangement of the reflection system (9) when the patient support (5) is arranged at the out-bore position and to determine optimized reflection system arrangement information for optimizing an arrangement of the reflection system (9).

## Description

### FIELD OF THE INVENTION

The present invention relates to a medical imaging system, a method for operating a medical imaging system, a computer program product, a computer-readable medium and a use of a workflow camera system for optimizing an arrangement of a reflection system of a medical imaging system.

### BACKGROUND OF THE INVENTION

Medical imaging systems and in particular those comprising an examination bore typically have only limited access and view from the outside. To allow an inside view of a patient during examination, in-bore cameras or mirror arrangements could be employed. Depending on the type of system and the environmental circumstances, the in-bore camera and/or mirror, however, often have only a limited or distorted view to a patient or a measurement setup arranged in the examination bore, or even no view at all. Hence, it may occur that an examination setup of the patient is unsuitable for obtaining a proper view to the patient during examination, which may be only recognized when the patient already in an in-bore position, or which may not be recognized at all.

Thus, it is an object of the invention to provide an improved system and method, which addresses the above noted drawbacks of the prior art.

### SUMMARY OF THE INVENTION

The problem is at least partially solved or alleviated by the subject matter of the independent claims of the present disclosure, wherein further preferred embodiments are incorporated in the dependent claims.

The present invention relates to a medical imaging system.

The medical imaging system comprises a medical imaging device comprising an examination bore.

The medical imaging system further comprises a patient support, wherein the patient support is movable between an in-bore and an out-bore position.

The medical imaging system further comprises a workflow camera system arranged outside the examination bore. The workflow camera system is further adapted to monitor a patient support area outside the examination bore.

The medical imaging system further comprises a reflection system comprising at least one reflective surface. The reflection system is adapted to provide a line-of-sight between an area of interest and an external device arrangeable remotely to the area of interest to emit radiation to the area of interest and/or receive radiation from the area of interest during examination.

Further, the workflow camera system is adapted to detect an arrangement of the reflection system when the patient support is arranged at the out-bore position. Further, the workflow camera system is adapted to determine optimized reflection system arrangement information for optimizing an arrangement of the reflection system.

Thus, an improved medical imaging system could be provided, allowing for an optimized reflection system arrangement. An improved detection of, for instance, a patient physiology or activity during examination via an external device could be accordingly obtained in a simple and cheap manner. The workflow camera system may provide to a user a suitable arrangement information for each part involved in the examination for achieving an optimized measurement via the reflection system. In other words, as will be elaborated in detail further below, an optimal reflective surface configuration for each specific case considering arrangements of a patient, coils, accessories etc. may be achieved. The employment of a workflow camera system may accordingly allow a simple and easy to implement solution to ensure a proper arrangement of the reflection system.

The medical imaging device may be a device generally capable of acquiring one or more images of a portion or a whole patient. The medical imaging device may be based on one or more of commonly known medical detection techniques, such as magnetic resonance, computer tomography (x-ray), positron emission, etc. Imaging may include the acquisition and/or reconstruction of one-dimensional measurement data, two-dimensional images, three-dimensional volumes and time-series of any aforementioned format.

The examination bore may be understood as an essentially tubular formed volume of the medical imaging device, wherein the patient or parts thereof are placed for examination. The examination bore may be accordingly sized and shaped to receive and house a whole patient or parts thereof during examination.

The patient support may have a bed-like appearance and may be sized and shaped such that a patient could be placed on it in a lying position, which may be a horizontal position. The patient support may be movable along an axis such that at least part of it could be moved into and out of the examination bore. An in-bore position of the patient support may be accordingly understood as a position, at which the patient support or portions thereof is arranged in the examination bore. Accordingly, an out-bore position of the patient support may be accordingly understood as a position, at which the patient support or portions thereof is arranged outside the examination bore. In some embodiments, an in-bore position may be a position in which the patient support is fully received by the examination bore. In some embodiments, out-bore position may be a position in which the patient support is fully arranged outside the examination bore.

The workflow camera system may be a system comprising one or more cameras or detectors that are suitable to acquire images or detect spatial or other information, e.g. spectral information. The workflow camera system may be generally used to guide and automate a patient examination setup and preparation. The workflow camera system may comprise a single camera. However, it will be understood that also a plurality of same or different cameras and/or sensors or detectors may be provided in the workflow camera system, dependent on the needs of the detection to be performed. The workflow camera system may be suitable to detect a workflow of an imaging procedure, which may include for instance a positioning of a patient on the patient support and arrangement of any further elements necessary for examination. One example for such further elements are magnetic resonance measurement coils in case the medical imaging device is a magnetic resonance imaging device. The patient support area may be the area in front of a medical imaging device, at which the patient support is located, when it is arranged at an out-bore position.

The reflection system may be understood as a system that may allow to guide a beam of electromagnetic radiation towards to and/or away from an area of interest (e.g. of the patient) during examination. In other words, a line-of-sight may be established between a first location inside, at the edge or outside the examination bore and the second location inside the examination bore, wherein a line-of-sight between the first and the second location would otherwise not exist. Thus, the reflection system may accordingly enable to observe an area of interest in the examination bore during examination, for instance from outside of the examination bore, which otherwise would be not observable.

The reflection system may include one or more reflective surfaces. The reflective surfaces may be suitably configured to reflect the electromagnetic radiation of a desired spectrum. The reflective surface may be for instance a mirror. In case an image of a portion of the patient should be reflected towards a camera arranged at the first location, the reflective surface may be suitably adapted to reflect visible light. The reflective surface may particularly provide a straight image-preserving optical path. However, the reflective surface is not delimited thereto and may be, for instance, adapted to reflect infrared light, ultraviolet light and/or any electromagnetic radiation of any other desired wavelength. The reflective surface may be completely or partially flat or curved and may be shaped in a rectangular, oval or any other desired shape.

The area of interest may include a patient's body or portions thereof of any desired size and shape. The reflection system may be adapted to reflect a single area of interest or simultaneously multiple areas of interest. The area of interest may be accordingly chosen such that suitable information could be derived from observing said area, such as physiological parameters of a patient, e.g. a breathing state etc. The area of interest may be, for instance, a part of the skin of a patient that can be used for plethysmography methods to detect a heart rate.

The external device may be a camera or sensor to receive and acquire image information from the area of interest via the reflection system. In some embodiments, the external device may be a projector or display, which may emit corresponding image information, which then could be presented to a patient inside the examination bore via the reflection system.

The workflow camera system may allow to detect an arrangement of the reflection system when the patient support is arranged at the out-bore position. Thus, the workflow camera system may acquire a positioning and arrangement of the elements of the reflection system and its allocation with respect to a patient. Also information about further elements arranged at the patient support may be acquired. The workflow camera system may detect an extension or orientation of the reflective surface. Considering the location and arrangement of the external device, the workflow camera system may determine if an improper arrangement is provided and may accordingly warn a user that no proper line-of-sight is or will be established during the examination between the external device and the area of interest.

The workflow camera system may accordingly suggest adjustments to a user with respect to the reflection system to obtain a proper line-of-sight. The workflow camera system may detect also further random parts that may influence a detection via the reflection system. Thus, the workflow camera system may detect the arrangement and shape of further parts of the reflection system, such as for mounting means for the reflective surface. The workflow camera system may accordingly determine if said parts would interfere with the examination bore or other elements of the medical imaging device, when the patient support would be moved, e.g. raised or lowered, or moved in or out of the examination bore. The workflow camera system may accordingly provide optimized reflection system arrangement information, which may include suggestions for an improved positioning and orientation of the monitored elements.

By adjusting the arrangement of the reflection system to an optimized condition, also a signal-to-noise ratio (SNR) of a measured signal from the area of interest may be improved. Generally, the relative position of the area to be imaged, the camera and the mirror may be high of importance. Hence, the better the positioning of the reflection system, the more signal may be potentially gained. For instance, the reflective element may be accordingly tilted such that a signal measured by the external device is maximized. The workflow camera system may accordingly comprise respective data processing means, which may include suitable software comprising for instance an algorithm that may allow to calculate based on the acquired information optimal mirror positions and configurations and/or an algorithm that may rate a mirror fixation or a mirror condition. Likewise, respective algorithms may be employed to estimate other potential signal generating areas based on known or measured object properties and materials or to determine potential noise generating surfaces to exclude from signal generation.

To determine the optimized reflection system arrangement information, the workflow camera system may consider one or more of a calibrated fixed camera position and angulation, known optical lens parameters of the camera or sensor employed in the workflow camera system, known shape and motion degrees of freedom of the one or more reflective surfaces, or known shape and motion degrees of freedom of all known coils or accessories further provided in the measurement setup.

The workflow camera system may be arranged above the patient support when arranged at the out-bore position such that the workflow camera system may face downward to detect the patient support area. Thus, a comprehensive overview about the full measurement set-up may be acquired in a top view image. Any parts or persons involved in a subsequent in-bore examination may be accordingly properly detected and considered when determining an optimized arrangement of the reflection system.

Some embodiments may additionally include an in-bore camera, as well, which may be used to additionally verify a correct positioning of the patient or other elements inside the bore. Such an in-bore camera may make use of a proper setup for acquiring patient images or general signals in the wavelength range 300 nm to 20000 nm. In an example, in-bore camera may be located at a first location, which may be an edge or flare of the examination bore, wherein the in-bore camera may be employed to detect a second location inside the examination bore, which may be for instance a patient's facial skin. The reflection system may accordingly provide a suitable optical path between the in-bore camera and the location to be detected.

An arrangement above the patient support may not be construed to an arrangement strictly perpendicular above a centerline of the patient support. Quite to the contrary, also inclined arrangements above the patient support may be provided. For instance, when multiple cameras or detectors are provided in the workflow camera system, one camera may be arranged perpendicular to a centerline of the patient support and further cameras or detectors may be arranged in an inclined manner or offset the centerline of the patient support, as desired. Accordingly, a "facing downward" of the workflow camera system may not be construed to a view only perpendicular to the patient support but also inclined top views may be encompassed.

The field of view of the workflow camera system may be suitably adapted to cover the whole patient support when arranged in an out-bore position. In case multiple cameras or sensors are employed, the respective multiple field of views may be combined by respective data processing means.

In a preferred embodiment, the optimized reflection system arrangement information includes information about a position, an angulation and/or a shape of the reflective surface.

Thus, an improved arrangement of the reflection system could be obtained. This may allow to determine and prevent a blocked line-of-sight between the area of interest and the external device. Furthermore, an improved SNR may be achieved for the external device. Furthermore, safety of the whole workflow for examination may be improved since any undesired interaction of parts of the reflection system with the patient or elements of the medical imaging device could be forecasted and accordingly prevented. Further, an improper mirror fixation or mirror contamination, degradation or damage could be determined and accordingly corrected. The optimized reflection system arrangement information may be suitably processed, e.g. via a respective digital processing means. For instance, a user may be provided with suggestions, e.g. via a display, for adjustments of an amended position and/or angulation of the reflective surface. In other words, a digital representation of the reflective surface and its environment may be acquired and optimized with respect to the intended line-of-sight of the external device with the area of interest.

In a preferred embodiment, the reflection system comprises a mounting system for mounting the reflective surface, and the optimized reflection system arrangement information includes information about a size and an arrangement of the mounting system.

Thus, a reliable arrangement of the reflective surface may be achieved. The mounting system may comprise one or more legs or bars, which could be removably attached to the patient support to allow an arrangement of the reflective surface at the patient support. In some embodiments a plurality of legs may be hingedly attached to each other. The mounting system arranged above the patient support may allow establishing a certain distance and angulation of a reflective surface with respect to a patient arranged on the patient support. The one or more legs may be provided, e.g. hingedly, at each lateral side of the patient support extending essentially upward from the patient support. Furthermore, one or more legs may be hingedly attached to the upwards facing legs and may extend essentially perpendicular thereto, i.e. essentially parallel to a top surface of the patient support, thus forming a bridge between the legs across the patient support. The reflective surface may be arranged at a suitable position at the one or more legs such that a line-of-sight may be established between an area of interest inside an examination bore, such as a portion of a patient, and the external device. The above noted arrangement with one or more legs is merely exemplarily, and it is to be understood that the mounting system may also be provided in a completely different manner by one or more support elements in any desired shape and orientation. The mounting system may be sized and shaped such that it can be completely received by the examination bore during examination. For instance, the legs of the mounting system may be adjustable in an extension direction, i.e. they can be lengthened or shortened, as desired. The optimized reflection system arrangement information may accordingly include suitable optimal lengths, orientations, angulations etc. for each element of the mounting system to allow a user to perform adjustments for achieving an optimized positioning of the reflection system.

In a preferred embodiment, the workflow camera system is adapted to detect at least a portion of a patient arranged at the patient support, preferably including a current position of the patient, a pose of the patient and/or a position of relevant body parts of the patient.

Thus, a proper detection of a target portion of the patient may be safeguarded to allow an improved investigation of said target portion by the external device during examination in the examination bore. The current position may include a location and spatial extension of the patient located on the patient support. A pose of the patient may include an orientation of one or more body parts of the patient on the patient support. The position of relevant body parts may include an arrangement of body areas to be investigated by the external device during examination in the examination bore. The optimized reflection system arrangement information may accordingly include adjustments to the position or pose of the patient. Also the safety of the patient may be enhanced, as it may be forecasted and ensured by the detection that no body parts may interfere with any elements of the reflection system or any further parts of the medical imaging device. For instance, it may be ensured that a relevant body part, which may be a body part that is to be investigated by the external device, is properly visible by providing a suitable line-of-sight, when the patient is arranged inside the examination bore.

In a preferred embodiment, the workflow camera system is adapted to detect an arrangement of at least one accessory arranged at the patient support including preferably at least a position, an angulation and/or a shape of the accessory.

Thus, a detection of one or more accessories may be provided to allow an improved investigation of an area of interest of a patient via the external device during examination in the examination bore. It could be particularly safeguarded, that no accessory is arranged in the line-of-sight between the area of interest and the external device. Hence, an improved signal may be obtained to be detected by the external device. The accessories may include, but are not limited to, measurement accessories such as magnetic resonance coils of patient related accessories, e.g. elements for positioning and stabilizing a patient during examination in the examination bore. The current position may include a location and spatial extension of the patient on the patient support. Also an angulation and/or a shape of the accessory may be considered. As will be appreciated, safety of the whole examination workflow may be improved since any undesired interaction of respective accessory with the patient or elements of the medical imaging device could be prevented during patient setup and the subsequent examination. A user may be provided with suggestions, e.g. via a display, for adjustments of the arrangement of the one or more accessories. In other words, a digital representation of the one or more accessories and may be acquired and optimized with respect to the intended line-of-sight with the external device.

In a preferred embodiment, the medical imaging system includes the external device, wherein the external device is preferably a physiology sensor arranged remotely to the area of interest, wherein the physiology sensor is adapted to detect an area of interest of a patient during examination.

Thus, one or more medical parameters of a patient may be determined at the imaging system during examination when a patient is arranged in the examination bore. The physiology sensor may be adapted to acquire respective information in form of image information allowing to determine one or more desired parameters, as will be explained further below. The physiology sensor may comprise an image sensor or a different kind of sensor that is suitable for acquiring information about a portion of interest of the patient during examination. For instance, a breathing state of the patient may be determined by imaging a respective chest or belly region of the patient. Generally, any kind of movement of the patient may be determined, such as for instance coughing or a tremor of the patient. Hence, additional technical and medical information is obtained that could be potentially combined with the data obtained from the regular examination of the medical imaging device. This may also improve the quality of the examination by the medical imaging device. For instance, poor or incorrect image data could be accordingly attributed to an occurrence of a corresponding unwanted movement of a patient.

A portion of interest may be a part of the body of a patient, for instance, the face, the chest or a different part from which respective physiological patient information could be acquired by the physiology sensor. The remote arrangement of the physiology sensor may include a distanced arrangement of the sensor to the medical imaging device. However, in some examples, the remote arrangement of the physiology sensor may also include an arrangement at the edge or even inside the medical imaging device.

The remote arrangement of the physiology sensor may allow a greater flexibility in the choice of sensors, which otherwise could not be provided in the medical imaging system due to space constraints or further physical constraints. For instance, a physiology sensor may be provided suitably remote to a magnetic resonance imaging system such that the physiology sensor is not influenced by the magnetic field surrounding the magnetic resonance imaging system. Furthermore, arranging the physiology sensor remote to the medical imaging device may allow an easy exchange and maintenance of said physiology sensor. Furthermore, the remote arrangement may allow to provide the physiology sensor in a non-sterile area, whereas the medical imaging device may be located in a sterile area.

In a preferred embodiment, the physiology sensor is configured to determine one or more of physiological parameter of a patient including one or more of cardiac signals, respiratory signals, skin perfusion signals, blood pressure signals, signals of an arrival of a bolus of injected agents, general motion of the patient, emotion of the patient, thermal signals.

Thus, the system may allow to flexibly determine one or more parameters when the patient is arranged in the examination bore that could otherwise not be monitored during examination. One or more physiological sensors may be provided that may be adapted to acquire the above noted one or more physiological parameter. In this respect, the physiological sensor may comprise a single detector, such as for instance an imaging detector, which may allow to acquire an image and from which image data a plurality of physiological parameters may be derived. However, also a plurality of different detectors may be provided at the physiology sensor that are each specialized to determine a distinct physiological parameter. To acquire the respective information, the physiology sensor may include one or more cameras or sensors that may operate in a wavelength range of 300 nm to 20000 nm. The determination of the one or more physiological parameters could be performed before, during and/or after the examination.

In a preferred embodiment, the external device additionally or alternatively includes a display or projector for providing image information to a patient arranged at the patient support.

Thus, image information could be provided to a patient, such as breathing guidance or patient entertainment. The provision of image data to a patient may accordingly also improve the imaging quality obtained by the examination. This not only accounts for providing breathing commands, which inherently improve the imaging conditions as the patient assumes a certain desired breathing state. During examination, a patient is also often requested to stay in a certain position or pose over a longer time without moving. However, children and anxious patients may nevertheless move due to discomfort or fear during the examination. Such fear could be diminished by distracting the anxious patient when an image or movie is presented to the patient during the examination. The display or projector may include any kind of suitable image transmitting device. The remote arrangement may accordingly allow to provide a display or projector that otherwise could not be provided in the vicinity of the medical imaging device for the reasons, as already explained above (e.g. strong magnetic fields etc.).

In a preferred embodiment, the workflow camera system includes one or more of an RGB-camera or a time-of-flight camera.

Thus, the desired information may be acquired by the different kind of cameras or sensors and potentially combined with each other. This may enhance the acquisition of the workflow camera further. For instance, an RGB camera may provide two-dimensional image information, whereas a time-of-flight camera may provide additional depth information, such that a three-dimensional representation of the observed area could be obtained. This may allow an improved detection of the patient support area outside the examination bore, which can be used to obtain a more sophisticated digital representation of the arrangement of the reflection system, the patient and potential further elements arranged on the patient support. Accordingly, also a data acquisition by the external device could be improved and the operational safety of the medical imaging system could be enhanced based on the optimized reflection system arrangement information.

In a preferred embodiment, the medical imaging system is a magnetic resonance imaging system or a computer tomography imaging system.

Thus, in addition to the image information acquired by the magnetic resonance imaging system or the computer tomography imaging system, an area of interest of the patient arranged in the examination bore could be suitably detected. The information of the respective magnetic resonance images or computer tomography images could be accordingly evaluated and combined with the additional information gathered from the external device. As will be appreciated, in particular magnetic resonance imaging systems and computer tomography imaging systems usually provide very limited additional space for further equipment or detectors. However, due to the optimized arrangement of the reflection system, an external device may be allowed to measure relevant physiological information of a patient during the examination in an optimized manner or a patient may be suitably provided with image data during the examination, as has been explained before in detail.

It is noted that the technology described herein could of course also be employed in other medical detection or imaging scenarios, such as for instance digital X-ray radiogrammetry (DRX), Image Guided Therapy (IGT) and/or radiotherapy (RT) etc.

The present invention also relates to a method for operating a medical imaging system.

The method comprises the step of providing a medical imaging system according to the present invention.

The method further comprises the step of arranging the patient support at the out-bore position.

The method further comprises the step of detecting the arrangement of the reflection system.

The method further comprises the step of determining reflection system arrangement information.

The method further comprises the step of optimizing an arrangement of the reflection system based on the determined reflection system arrangement information.

Thus, an improved method for operating a medical imaging system may be provided, allowing for an optimized reflection system arrangement. An improved detection of, for instance, a patient physiology or activity during examination via an external device could be accordingly obtained in a simple to implement and cheap manner. The workflow camera system may provide a user with potential adjustments for arrangements of each part involved in the examination to achieve an optimized measurement via the reflection system.

Generally, the features and advantages explained herein with respect to the medical imaging system according to the present invention similarly apply to the method for operating said medical imaging system.

In a preferred embodiment, the method further comprises the step of providing a line-of-sight between an area of interest inside the examination bore and an external device arranged remotely to the area of interest to emit radiation to the area of interest and/or receive radiation from the area of interest during examination.

Thus, an improved method to resolve and prevent a blocked line-of-sight between the area of interest and the external device could be provided. Also, an improved SNR for the external device may be achieved, as already explained above. Likewise, safety of the whole workflow for preparation and examination may be improved because any undesired interaction of respective parts of the reflection system with the patient or elements of the medical imaging device could be prevented.

The present invention also relates to a computer program product comprising instructions to cause, when executed by a computer, the medical imaging system according to the present invention to execute at least the steps of determining reflection system arrangement information and/or optimizing an arrangement of the reflection system based on the determined reflection system arrangement information of the method of the present invention.

The features of the system according to the present invention may be implemented by respective suitable digital or computational means, which may include, for instance, one or more computers, apps and/or networks.

The method may be at least partly computer-implemented, and may be implemented in software or in hardware, or in software and hardware. The software may comprise respective algorithms that may provide an identification of all relevant parts of the examination setup from the acquired sensor or image data of the workflow camera and that may provide a forecast about future scenarios.

Corresponding calculations may be performed with different boundary conditions and loss functions, such as taking into account possible locations of a determined reflective surface mirror for a given type of examination and patient position. Additionally, a penalizing modification effort from a current setup could be considered with variable weights. In some embodiments, neural network keypoint detection networks may be employed. The keypoints may be designed such that they may provide condensed information about possible deformations and the available motion degrees of freedom of the elements detected. Keypoints may be for instance derived in 3D directly, by triangulation or with help of a depth camera.

The computing system or data processing means as described herein may be any suitable computing means, such as an electronic control module etc., which may be a localized or a distributed computer system. The data processing means or the computing system, respectively, may comprise one or more of a processor, a memory, a data interface, or the like. The software may also be employed to provide a distinction between known required equipment and unknown (random) further objects. This may be used to balance between different potential solutions for optimization by relocating a random object and adjustments of the mirror, while ensuring a proper position of a required coil, accessory or object.

The features and advantages outlined above in the context of the medical imaging system and the method similarly apply to the computer program product described herein.

The present invention also relates to a computer-readable medium having stored thereon the computer program product of the present invention.

Generally, the features and advantages explained herein with respect to the system and method according to the present invention similarly apply to the computer-readable medium having stored thereon the computer program product of the present invention for executing certain steps of the method.

A computer program may be stored/distributed on a suitable medium such as an optical storage medium or a solid-state medium, supplied together with or as part of other hardware, but may also be distributed in other forms, such as via the Internet or other wired or wireless telecommunication systems.

Any of the computer/computing system, the computer-program product and/or the computer-readable medium described herein may be at least included in the system of the present invention or may be at least operatively coupled thereto.

The present invention also relates to a use of a workflow camera system for optimizing an arrangement of a reflection system of a medical imaging system according to the present invention. The use is based on optimized reflection system arrangement information determined by the workflow camera system.

Thus, by the use of the workflow camera, an improved user guidance, e.g. during patient setup and subsequent examination could be provided. Hence, a patient and further elements may be optimally positioned when a medical examination, e.g. an imaging via a respective medical imaging device, is to be conducted. The employment of a workflow camera may also allow achieving a compatible setup with a planned patient setup and examination workflow. This may include, for instance, an accessibility of patient infusion sheaths or applicability of immobilization devices provided in the setup.

Generally, the features and advantages explained in this disclosure with respect to the medical imaging system and the method for operation said medical imaging system according to the present invention similarly apply to the use of a workflow camera system for optimizing an arrangement of a reflection system of a medical imaging system.

Further features, examples, and advantages will become apparent from the following detailed description of preferred embodiments and the accompanying figures.

### BRIEF DESCRIPTION OF THE DRAWINGS

For a better understanding of the present invention, and to illustrate its practicality, figures are provided in the following and reference is made thereto. It should be understood that the figures represent only exemplary embodiments and thus in no way limit the scope of the claimed invention. Any reference signs in the claims should not be construed as limiting the scope of the claims. The figures are merely schematic representations and serve only to illustrate examples of the disclosure. In the accompanying drawings:
Fig. 1 schematically illustrates an embodiment of a medical imaging system according to the present invention;
Fig. 2 schematically illustrates an embodiment of a medical imaging system according to the present invention;
Fig. 3 schematically illustrates an embodiment of a close-up view of a medical imaging system according to the present invention;
Fig. 4 schematically illustrates an embodiment of a method according to the present invention.

### DETAILED DESCRIPTION OF EMBODIMENTS

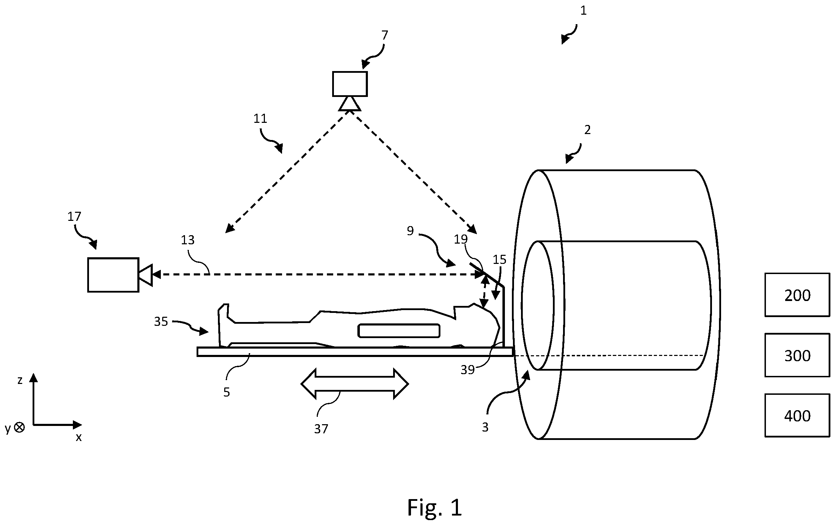

Fig. 1 schematically illustrates a medical imaging system 1 according to the present invention. The medical imaging system 1 includes a medical imaging device 2 comprising an examination bore 3. The examination bore 3 is sized and shaped to allow receiving a patient 35 for examination. The patient 35 is arranged on a patient support 5, wherein the patient support 5 is linearly movable along an x-axis between an in-bore and an out-bore position as indicated by the double arrow 37.

Fig. 1 shows the patient support 5 being arranged in an out-bore position. In the depicted embodiment, the patient support 5 is a table upon which the patient 35 lies. The depicted embodiment, the patient lies head first on the table towards the examination bore 3. However, the patient 35 may be arranged as desired dependent on an area or volume of interest to be examined by the medical imaging device 2. A workflow camera system 7 is arranged outside the examination bore 3 above the patient support 35 and adapted to acquire or monitor a patient support area 11 outside the examination bore 3. In the depicted embodiment, the workflow camera system 7 comprises one camera. However, it will be understood that also a plurality of (different) cameras and/or sensors may be provided, dependent on the needs of the detection to be performed. Further, a reflection system 9 is provided comprising at least one reflective surface 19. The depicted embodiment, the reflective surface 19 is a mirror, which is held in an angular manner by a mounting system 39. An external device 17 is arranged remotely to the medical imaging system 1.

The external device 17 is a physiology sensor in form of a camera. Even though the external device 17 as depicted comprises one camera, it will be understood that also a plurality of (different) cameras and/or sensors may be provided, dependent on the needs of the detection to be performed. The camera is configured to determine an image of the area of interest 15 of the patient 35, which is in the depicted embodiment a head region of the patient 35. However, it is apparent that also different regions of interest 15 of the patient 35 may be acquired dependent on the configuration of the reflective surface 19 and its arrangement with respect to the patient 35. The external device 17 may accordingly detect or image for instance a movement of a portion of the patient 35 at the area of interest 15 and may accordingly allow to determine one or more physiological parameters of the patient 35 including one or more of cardiac signals, respiratory signals, skin perfusion signals, blood pressure signals, signals of an arrival of a bolus of injected agents, general motion of the patient, emotion of the patient, thermal signals etc. Hence, the reflection system 9 provides a line-of-sight 13 between the area of interest 15 and allows to receive image information from the area of interest 15. In other words, a light beam is deflected from the area of interest 15 at the reflective surface 19 towards the external device 17, thus establishing an angularly deflected line-of-sight 13 between the area of interest 15 and the external device 17. In the depicted embodiment, the external device 17 is arranged on the left side, i.e. the patient side of the medical imaging device 2. However, the external device 17 may also be provided at the opposite side, i.e. at the right side of the medical imaging device 2. In this case, the reflective surface 19 could be accordingly angled to the opposite direction to enable a respective detection of the area of interest 15. In some embodiments, the external device 17 may also be arranged at an edge or even inside the medical imaging device 2, such as in the examination bore 3, as well. Also in this case, the reflective surface 19 could be accordingly arranged to enable a respective detection of the area of interest 15 via the external device 17.

It is apparent that in case the external device 17 is not a sensor, but for instance a radiation emitting device, such as a laser (or projector), respective radiation may be emitted from the external device 17 the towards the area of interest 15. Respective reflected radiation may be accordingly guided back along the line-of-sight 13 towards a respective sensor of the external device 17. Hence, an optical path is established between the area of interest 15 and the external device 17 generally allowing an optical two-way communication between the area of interest 15 and the external device 17. This is indicated by the double arrows indicating the line-of-sight 13. Above the patient support 5, a workflow camera system 7 is arranged.

The workflow camera system 7 is adapted to monitor a patient support area 11 outside the examination bore 3 and accordingly detects an arrangement of the reflection system 9 when the patient support 5 is arranged at an out-bore position, as depicted. In particular, the workflow camera system 7 faces downward to the patient support area 11. In other words, the patient support area 11 is viewed essentially from the top. However, in different embodiments, the workflow camera, may also be arranged at a different position, as desired, e.g. in an inclined manner or even from the front side, the back side or the lateral sides of the patient support area 11. The workflow camera system 7 may accordingly determine reflection system arrangement information by acquiring a respective position and/or orientation of the reflection system 9. The image information acquired by the camera 7 may then be provided to data processing means, such as the computer 200 for further data processing such as feature recognition and/or image reconstruction.

The reflection system arrangement information may be fed to the data processing means, which accordingly determines a current configuration and may calculate an improved optimized arrangement, which may be referred to as optimized reflection system arrangement information. This optimized information may then be provided to a user to allow him or her to adapt the current configuration to an improved configuration. Thus, the reflection system 9 may be accordingly optimized based on the optimized reflection system arrangement information determined by the workflow camera system 7.

The optimized reflection system arrangement information accordingly includes for instance information about a position, an angulation and/or a shape of the reflective surface 19. The optimized reflection system arrangement information may be evaluated by a data processing means, such as the computer 200, comprising a computer program product 300 and/or a computer readable medium 400, having data processing means, such as software stored, which is suitably adapted to evaluate the acquired information of the workflow camera system 7. This may include, for instance the processing of image data acquired from the workflow camera system 7, which may consider the arrangement of the reflection system 9. Hence, an optimized arrangement of the reflection system 9 may be suggested based on the determined reflection system arrangement information. The computer 200 may be part of the workflow camera system 7 or may be external and operatively coupled thereto. For instance, an optimal mirror of position may be determined and suggested to a user. The system may accordingly consider the line-of-sight 13 between the area of interest 15 inside the examination bore 3 and the external device 17, and the software may accordingly suggest suitable arrangements, e.g. an adjustment of a current position and/or an angulation of the reflective surface 19 to a user.

Accordingly, all relevant information for providing an optimized arrangement of the reflection system 9 may be collected by the computer 200, which may include one or more of a position or orientation of the external device 17, a respective line-of-sight 13 to an area of interest 15, a position, a size and a shape of the patient support 5 and a patient 35 arranged thereon, a size and a shape of the medical imaging device 2, a size and a shape of the examination bore 3, or an arrangement of potential measuring means of the medical imaging device 2, such as magnetic resonance coils arranged at the patient 35 (not depicted). Also, further information, such as further arbitrary devices and elements, which are typically arranged on the patient support 5 and which may influence the measurement via the external device 17 may be considered.

The optimization of the arrangement of the reflection system 9 is accordingly performed when the patient support 5 is in an out-bore position, as depicted. Furthermore, a user may be warned if the reflection system 9 has a configuration, wherein it may collide with the examination bore 3, for instance when the patient support 5 is subsequently moved into the examination bore 3 along the x-axis or moved in a horizontal manner, along the z-axis.

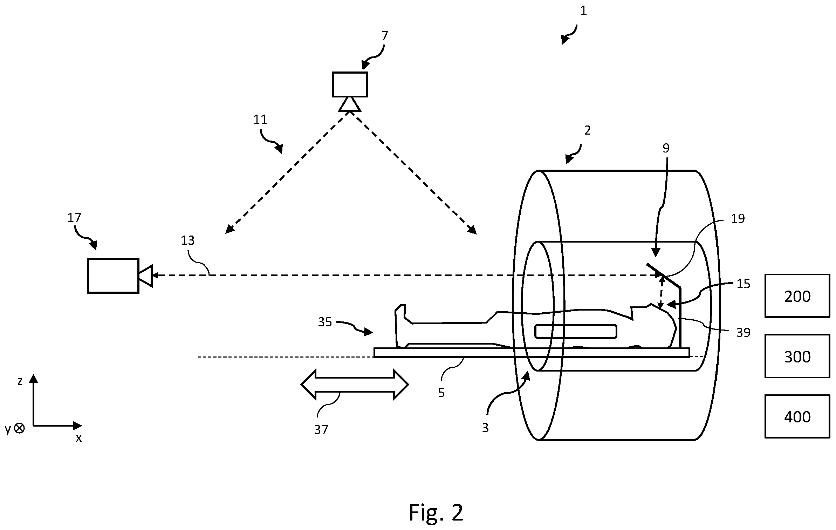

Fig. 2 schematically illustrates the medical imaging system 1 according to Fig. 1, when the patient support 5 is in an in-bore configuration. That is, the patient support 5 has been moved along the x-axis until the patient 35 has arrived at a desired position in the examination bore 3. As depicted, the external device 17 has a line-of-sight 13, which is achieved by an optimized arrangement of the reflection system 9. The optimization may accordingly consider the position of the area of interest 15 when the patient support 5 is in an in-bore position. Any relevant elements that were previously acquired by the workflow camera system 7, when the patient support was at the out-bore position, have been considered to provide an optimized arrangement of the reflection system 9. Thus, the external device 17 is enabled to measure a physiological parameter of the patient during examination of the medical imaging device 2 in an undisturbed manner. In a different embodiment, the external device 17 is a projector or display, which creates an image that is accordingly reflected to the patient 35 via the line-of-sight 13. It is also ensured by the optimized positioning arrangement of the reflection system 9, that no undesired interference could occur with any parts of the medical imaging device 2 when the patient support 5 is moved with respect to the medical imaging device 2. In a further embodiment, when the external device 17 is an external physiological sensor that is adapted to optically detect a certain parameter at the patient, and when it is determined that an SNR of that sensor is low, the workflow camera system 7 may accordingly suggest an improvement of the arrangement of the reflective surface 19 to increase an SNR and accordingly improve the detection by the external physiological sensor.

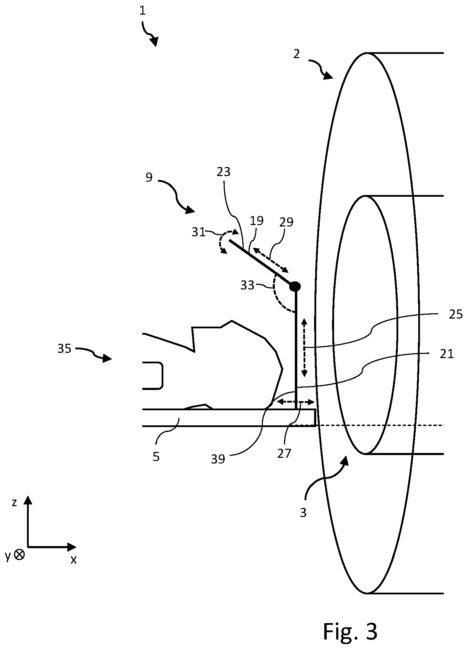

Fig. 3 schematically illustrates an enlarged view of the medical imaging system 1 according to Fig. 1 when the patient support 5 is an out-bore position. As described before, the workflow camera system 7 has acquired the arrangement of the reflection system 9, which arrangement may be accordingly optimized based on the determined reflection system arrangement information. The depicted embodiment, the reflection system 9 includes a mounting system 39 for the reflective surface 19 comprising a first leg 21 mounted on the patient support 5. Hingedly attached to the first leg 21 is a second leg 23, wherein the second leg 23 may be rotated around an angle 33. The first leg 21 is adjustable in the X or Z direction. In other embodiments, more than one first leg 21 and more than one second leg 23 may be provided. For instance, two first legs 21 may be provided at each lateral side of the patient support 5 and a single second leg 23 may extend between the two first legs 21 such that the second leg 23 forms a bridge across the patient support 5. Each leg may be adjustable in a length and/or an angular orientation with respect to the patient support 5.

In the depicted embodiment, the first leg 21 may be adjustable in a height direction (z-axis) via a first movement 25 and a second movement 27 along a longitudinal direction (x- axis). The second leg 23 may be adjusted in length as depicted by arrow 29 and may be rotated in an x-z-plane about an angle 33, as depicted by arrow 31. It is obvious, that the depicted movements of the legs are merely exemplary and that the legs may also be allowed to be moved or adjusted additionally and/or alternatively perpendicular to the x-z-plane (y-axis), as desired. In the depicted embodiment, the reflective surface 19 is attached to the second leg 23. Thus, considering the optimized reflection system arrangement information of the workflow camera system 7, the reflective surface 19 could be properly adjusted with respect to a line-of-sight 13 between an area of interest 15 and an external device 17 (cf. Figs. 1 and 2). Furthermore, potential collisions between parts of the reflection system 9 and the medical imaging device 3 may be prevented. For instance, if an angle 33 or a length of the second leg 23 are too large, a distal end of the second leg 23 may collide with an inner surface of the examination bore 3, when the patient support 5 is moved into the examination bore 3. The workflow camera system 7 may now detect that the angle 33 is undesirably large and may accordingly provide the information that the angle 33 should be reduced or that the second leg may be suitable moved along arrow 29 such that the distal tip no longer collides. Or, in a different example, the tilting angle 33 does not provide a proper line-of-sight 13 between an area of interest 15 and an external device 17. In this scenario, the workflow camera system 7 may warn a user that the angle 33 is not properly adjusted and may suggest an amendment of the angle 33. In this respect, the workflow camera system 7 may also forecast the configuration when the patient support 5 is moved into the examination bore 3.

In a further scenario, one or more elements of the reflection system 9 may extend beyond the patient support 5 in the x-direction, such that a lowering or raising of the patient support 5 in a z-direction would lead to a collision with the medical imaging device 2. The workflow camera system 7 may now detect that the parts of the reflection system 9 are not safely arranged and may issue a warning to a user and/or propose a respective amendment of the arrangement of the elements of the reflection system 9, such that the patient support 5 could be safely raised or lowered.

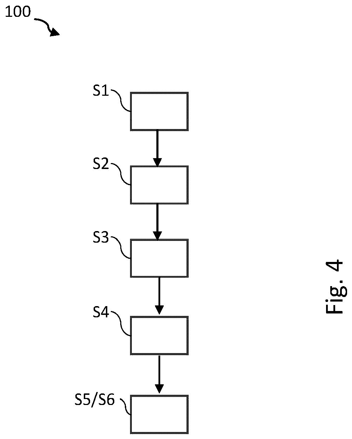

Fig. 4 schematically illustrates an embodiment of a method 100 according to the present invention. The method 100 includes the step S1 of providing a medical imaging system 1 as described herein. Further, the method 100 includes the step S2 of arranging the patient support 5 at the out-bore position. Further, the method 100 includes the step S3 of detecting the arrangement of the reflection system 9. Further, the method 100 includes the step S4 of determining reflection system arrangement information. In step S5, based on the determined reflection system arrangement information, a user may optimize the arrangement of the reflection system 9. This may be done for instance by determining potential adjustments to the currently monitored configuration. A user may be accordingly supported by respective software that may suggest amendments to the present configuration of the reflection system 9. As depicted, the method also includes the step S6 of providing a line-of-sight 13 between an area of interest 15 inside the examination bore 3 and an external device 17 arranged remotely to the area of interest 15 to emit radiation to the area of interest 15 and/or receive radiation from the area of interest 15 during examination.

Other variations to the disclosed embodiments can be understood and effected by those skilled in the art in practicing the claimed disclosure, from the study of the drawings, the disclosure, and the appended claims. In the claims the word "comprising" does not exclude other elements or steps and the indefinite article "a" or "an" does not exclude a plurality. The mere fact that certain measures are recited in mutually different dependent claims does not indicate that a combination of these measures cannot be used to advantage.

### LIST OF REFERENCE SIGNS

- 1: medical imaging system
- 2: medical imaging device
- 3: examination bore
- 5: patient support
- 7: workflow camera system
- 9: reflection system
- 11: patient support area
- 13: line-of-sight
- 15: area of interest
- 17: external device
- 19: reflective surface
- 21: first leg
- 23: second leg
- 25: first movement
- 27: second movement
- 29: third movement
- 31: fourth movement
- 33: angle
- 35: patient
- 37: patient support movement
- 39: mounting system
- 100: method
- S1-S6: method steps
- 200: computer
- 300: computer program product
- 400: computer-readable medium

## Claims

1. A medical imaging system (1) comprising:
a medical imaging device (2) comprising an examination bore (3),
a patient support (5), wherein the patient support (5) is movable between an in-bore and an out-bore position,
a workflow camera system (7) arranged outside the examination bore (3) and adapted to monitor a patient support area (11) outside the examination bore (3),
a reflection system (9) comprising at least one reflective surface (19), wherein the reflection system (9) is adapted to provide a line-of-sight (13) between an area of interest (15) and an external device (17) arrangeable remotely to the area of interest (15) to emit radiation to the area of interest (15) and/or receive radiation from the area of interest (15) during examination,
wherein the workflow camera system (7) is adapted to detect an arrangement of the reflection system (9) when the patient support (5) is arranged at the out-bore position and to determine optimized reflection system arrangement information for optimizing an arrangement of the reflection system (9).

2. The medical imaging system (1) according to the preceding claim,
wherein the optimized reflection system arrangement information includes information about a position, an angulation and/or a shape of the reflective surface (19).

3. The medical imaging system (1) according to one of the preceding claims,
wherein the reflection system (9) comprises a mounting system (39) for mounting the reflective surface (19), and
wherein the optimized reflection system arrangement information includes information about a size and an arrangement of the mounting system (39).

4. The medical imaging system (1) according to one of the preceding claims,
wherein the workflow camera system (7) is adapted to detect at least a portion of a patient arranged at the patient support (5), preferably including a current position of the patient, a pose of the patient and/or a position of relevant body parts of the patient.

5. The medical imaging system (1) according to one of the preceding claims,
wherein the workflow camera system (7) is adapted to detect an arrangement of at least one accessory arranged at the patient support (5) including preferably at least a position, an angulation and/or a shape of the accessory.

6. The medical imaging system (1) according to one of the preceding claims,
wherein the medical imaging system (1) includes the external device (17),
wherein the external device (17) is preferably a physiology sensor arranged remotely to the area of interest (15),
wherein the physiology sensor is adapted to detect an area of interest (15) of a patient (35) during examination.

7. The medical imaging system (1) according to the preceding claim,
wherein the physiology sensor is configured to determine one or more of physiological parameter of a patient (35) including one or more of cardiac signals, respiratory signals, skin perfusion signals, blood pressure signals, signals of an arrival of a bolus of injected agents, general motion of the patient (35), emotion of the patient (35), thermal signals.

8. The medical imaging system (1) according to claim 6 or 7,
wherein the external device (17) additionally or alternatively includes a display or projector for providing image information to a patient (35) arranged at the patient support (5).

9. The medical imaging system (1) according to one of the preceding claims,
wherein the workflow camera system (7) includes one or more of an RGB-camera or a time-of-flight camera.

10. The medical imaging system (1) according to one of the preceding claims,
wherein the medical imaging system (1) is a magnetic resonance imaging system or a computer tomography imaging system.

11. A method for operating a medical imaging system (1), comprising the steps of:
providing (S1) a medical imaging system (1) according to one of the preceding claims 1 to 10,
arranging (S2) the patient support (5) at the out-bore position,
detecting (S3) the arrangement of the reflection system (9),
determining (S4) reflection system arrangement information,
optimizing (S5) an arrangement of the reflection system (9) based on the determined reflection system arrangement information.

12. Method according to the preceding claim, further comprising:
providing (S6) a line-of-sight (13) between an area of interest (15) inside the examination bore (3) and an external device (17) arranged remotely to the area of interest (15) to emit radiation to the area of interest (15) and/or receive radiation from the area of interest (15) during examination.

13. A computer program product (300) comprising instructions to cause, when executed by a computer 200, the medical imaging system of any one of claims 1 to 10 to execute at least the steps of determining (S4) reflection system arrangement information and/or optimizing (S5) an arrangement of the reflection system (9) based on the determined reflection system arrangement information of the method of claim 11.

14. A computer-readable medium (400) having stored thereon the computer program product (300) of claim 13.

15. Use of a workflow camera system (7) for optimizing an arrangement of a reflection system (9) of a medical imaging system (1) according to one of the preceding claims 1 to 10 based on reflection system arrangement information determined by the workflow camera system (7).
